Europäisches Patentamt

(19) European Patent Office

Office européen des brevets

(11) Publication number : **0 451 130 A2**

(12) **EUROPEAN PATENT APPLICATION**

(21) Application number : 91870055.0

(22) Date of filing : 04.04.91

(51) Int. Cl.⁵ : **A61K 31/495**

(30) Priority : 05.04.90 US 504584

(43) Date of publication of application :
09.10.91 Bulletin 91/41

(84) Designated Contracting States :
AT BE CH DE DK ES FR GB GR IT LI LU NL SE

(71) Applicant : BALTIMORE BIOTECH, INC.
St. Joeseph's Hospital, 7620 York Road
Towson, Maryland 21204 (US)

(72) Inventor : Glaser, Bert M.
11201 Valley Heights Drive
Owings Mills, Maryland 21117 (US)
Inventor : Varner, Hugh H.
318 Ringold Valley Circle
Cockeysville, Maryland 21030 (US)

(74) Representative : Colens, Alain
Rue F.Merjay, 21
B-1060 Brussels (BE)

(54) Use of amiloride and other pyrazine derivatives for preventing or treating ocular neovascularization.

(57) A method and composition for preventing and/or treating ocular (including corneal, iridial, retinal, and choroidal neovascularization) in a patient comprises administration of a compound in the class of pyrazine derivative, e.g. amiloride, or one of its derivatives or congeners or one of their pharmaceutically acceptable salts and/or solvates, to the patient. The pyrazine derivatives can be administered by topical administration to ocular tissues with a suitable carrier such as an artificial tear carrier or a petroleum-based ointment, among others. The pyrazine derivative can also be administered orally, by intravenous administration, or by an intraocular implant.

EP 0 451 130 A2

## Field of the Invention

The present invention relates to the field of treating ocular tissues, and more particularly to treating ocular tissues to prevent neovascularization. More specifically, the present invention relates to preventing corneal neovascularization, iris neovascularization, retinal neovascularization, and choroidal neovascularization.

## Background of the Invention

In certain pathological conditions of the eye, and as a result of certain eye treatments or traumatic insult to the eye, the formation of blood vessels in ocular tissues often occurs. Such vessel formation is known as neovascularization, and neovascularization is often an unwanted and undesirable occurrence.

One example of undesirable ocular neovascularization is neovascularization occurring in corneal tissues. Corneal neovascularization can arise following any inflammatory or traumatic insult to the cornea. Chief among such causes is corneal transplantation. However, corneal neovascularization also occurs following herpes keratitis which is a fairly common infectious corneal lesion. In addition, corneal neovascularization can occur after any corneal trauma such as might occur following automobile accidents, athletic injury, etc. and can lead to clouding of the cornea and loss of vision.

Further, with respect to corneal transplantation, when corneal neovascularization occurs after corneal transplantation, it often precipitates graft rejection. Generally speaking, the corneal graft is usually considered as not susceptible to rejection processes of the patient because there is normally no blood supply serving the corneal tissue. Therefore, ophthalmologists have been in the unique position of not needing to perform tissue matches prior to doing corneal transplants. However, once corneal neovascularization occurs, then a tissue match becomes imperative to avoid tissue rejection, making the process of corneal transplantation much more complex, especially if a tissue rejection subsequently takes place. Once the cornea is rejected, another transplant may be necessary with an increased risk of future rejection. This problematic situation is particularly significant in the case of corneal transplantation because the recovery time following the corneal transplant is often as long as six to eight months before useful vision can be achieved.

In cases of corneal neovascularization where graft rejection is not a risk, i. e. after trauma or inflammation, the corneal blood vessels tend to induce localized scarring and tissue edema resulting in cloudiness of the normally clear cornea. This cloudiness significantly impedes vision.

Although data is not highly precise, it is conservatively estimated that approximately 36,000 persons in the United States are at risk each year to corneal neovascularization. This number is extrapolated from the number of corneal transplants done per year. Thus, approximately 36,000 people each year are subject to or at risk of impeded vision and loss of the ability to perform their jobs adequately due to corneal neovascularization. Thus, corneal neovascularization is seen as both a severe medical and a severe economic problem.

Another instance of undesirable ocular neovascularization is neovascularization of retinal tissues. Neovascularization from the inner surface of the retina occurs secondary to diabetic retinopathy, retinal branch vein occlusions, retinal central vein occlusions, sickle cell retinopathy, retinopathy of prematurity, and other various and sundry retinopathies.

Retinal neovascularization has been recognized as a problem for quite some time. Since the discovery of insulin in the 1940's, the longevity of diabetics has increased significantly, thereby resulting in an escalation of the amount of diabetic retinopathy diagnosed, since the occurrence of diabetic retinopathy is directly related to the duration of the diabetes. Most diabetics, by the time they have had diabetes for ten to fifteen years, have some form of diabetic retinopathy, and, by twenty years, almost all diabetics have significant diabetic retinopathy which interferes with vision. There are approximately 50,000 new cases of diabetic retinopathy each year in the United States.

Iris neovascularization is often associated with retinal neovascularization and results in untreatable glaucoma.

In contrast, neovascularization growing from the choroid under the retina, commonly called choroidal neovascularization, occurs in a different group of diseases. These include mainly age-related macular degeneration, histoplasmosis, various forms of ocular trauma, and a host of other chorioretinal disorders. Chorioretinal disorders also lead to vision impairment of different degrees and can lead to blindness. There are probably about 200,000 new cases of age-related macular degeneration per year in the United States.

Furthermore, neovascularization from the choroid under the retina as occurs in age-related macular degeneration and neovascularization from the surface of the retina as occurs in diabetic retinopathy are the major causes of blindness in the industrialized world. It would be desirable, therefore, to provide an ocular treatment that prevents neovascularization that occurs in retinal and choroidal tissues.

The only treatment currently available for treating corneal neovascularization is to treat the cornea with anti-inflammatory agents, most commonly steroids, to reduce any inflammatory component inducing the neovascular response.

As far as treatments for retinal neovascularization are concerned, pan retinal photocoagulation is employed. In this treatment, chorioretinal scars are induced with a laser in the retinal periphery in a scattered pattern. It appears that the scar tissue inhibits neovascularization. More specifically, the cells and materials in the scar tissue have been shown to include numerous pigment epithelial cells, and it has been learned that a pigment epithelial cell-derived protease inhibitor (PEPI) is important in inhibition of the potent protease urokinase-like plasminogen activator or uPA. This treatment seems to be effective in most cases and reduces the risk of blindness by about half. It would certainly be desirable, however, to have a treatment to prevent retinal neovascularization that is effective in more than only half of the cases.

An undesirable side effect of the retinal scarring is the destructive effect it has on the patient's peripheral and night vision. It would certainly be desirable to provide a treatment for retinal neovascularization that avoids impairment of the patient's peripheral and night vision.

With respect to choroidal neovascularization, this condition is treated with direct laser treatment to obliterate the new blood vessels stemming from the choroid. Unfortunately, this treatment method is best performed when the blood vessels are outside of the foveal avascular zone. In a study performed in the 1980's, it was determined that only 20% of patients with choroidal neovascularization secondary to age-related macular degeneration had the lesion outside the foveal avascular zone. Since treatment of the foveal avascular zone results in the destruction of the cones in the fovea in this area, direct laser treatment itself would cause loss of vision. Therefore, only 20% of all patients were eligible for the direct laser treatment. Of those treatable patients, approximately 50% are helped by laser treatment for the first one to two years. However, of that group that was initially treatable, 60% to 70% have recurrences of the choroidal neovascularization, and the recurrences are usually not amenable to further treatment. It is clear, therefore, that the present treatment for choroidal neovascularization secondary to age-related macular degeneration is woefully inadequate.

The scientific literature discloses that a number of materials have shown the ability to inhibit neovascularization in certain circumstances. In Invest. Ophthalmol. Vis. Sci., Vol. 22, pg. 191-9, 1982, "Plasminogen Activator (Urokinase) Causes Vascularization of the Cornea", by Berman et al, there is a disclosure that chloromethyl ketone Phe-Ala-ArgCH$_2$Cl-2HCl inhibits rabbit uPA in rabbit corneas and provides some degree of inhibition of neovascularization. In Seminars in Thrombosis and Hemostasis, Vol. 12, No. 4, 1986, "Plasminogen Activators (Urokinase) Mediate Neovascularization: Possible Role in Tumor Angiogenesis", by Goldfarb et al, there is a disclosure that 3H diisopropylfluorophosphate ($^3$H-DFP) inhibits rabbit uPA in rabbit corneas providing some degree of neovascularization inhibition. In Invest. Ophthalmol. Vis. Sci., Vol. 29, P. 109, 1988, "A Urokinase Inhibitor Derived from Human RPE Cells Inhibits Neovascularization in Vivo", by Glaser, Jerdan, et al, there is a disclosure that purified pigment epithelial protease inhibitor (PEPI) inhibits rabbit uPA in rabbit corneas and provides some inhibition of neovascularization. In Business Week, January 22, 1990 edition, "This Protein May Strangle Tumors", by Marbach, there is a disclosure that the natural protein called platelet factor 4 inhibits unspecified materials in unspecified animals, providing some degree of inhibition of blood vessel formation. In Science, Vol. 247, pg. 77-9, 1990, "Inhibition of Angiogenesis by Recombinant Human Platelet Factor-4 and Related Peptides", by Maione et al, there is a disclosure that this factor inhibits human vascular endothelial cell division in vitro. In Science, Vol. 235, pg. 442-47, 1987, "Angiogenic Factors", by Folkman et al there is a disclosure that a mixture of heparin and cortisone inhibits neovascularization with respect to tumor growth. Other materials inhibiting neovascularization in tumor growth include copper and oxygen.

All of the materials mentioned above that have a property of inhibiting neovascularization have problems or other undesirable features. For example, PEPI is a relatively large molecule and, as such, is relatively difficult to diffuse through ocular tissues. PEPI is a natural product and may be very expensive to isolate and synthesize. As a large molecule, PEPI may initiate an undesired immune response.

Turning to the DFP material, this a general serine protease inhibitor which is not selective as to uPA. DFP is used routinely to inhibit a wide gamut of enzymes including trypsin and chymotrypsin, among others. Moreover, DFP is extremely toxic and is not a very good inhibitor. It takes a relatively long time to bind to the enzyme and is needed in large concentrations.

With respect to the uPA inhibitor disclosed in the Berman et al article, the inhibitor required high concentrations, prolonged incubations, and repeated applications to be effective. These inhibitors have little potential as therapeutic agents because of probable toxicity due to their ability to alkylate various cellular nucleophiles.

It would be desirable, therefore, to provide a material to inhibit ocular neovascularization that is commonly available, is non-toxic, is specific, and whose pharmacological properties and side effects are well known.

The material known as amiloride, in particular the monohydrochloride dihydrate of amiloride, is a commonly available material whose pharmacological properties and side effects are well known. Amiloride monohydrochloride dihydrate is well known as a diuretic that is administered orally.

The scientific literature also discloses the use of amiloride in several other contexts. In FEBS Letters, Vol. 214, pg. 187-191, by Vassalli and Belin, amiloride is discussed in relation to the inhibition of human uPA catalyzed hydrolysis of a thiobenzyl benzyloxycarbonyl-L-lysinate substrate in vitro. The article of Vassalli and Belin additionally, discloses that amiloride competitively inhibits the catalytic activity of uPA on plasminogen and of uPA on the serpin family of macromolecular antiproteases in vitro without decreasing the activity of either tissue-type plasminogen activator (tPA) or of plasmin. However, the authors conclude by suggesting a further study to include amiloride analogs and suggest that amiloride itself may not be specific enough to be useful as an anti- uPA agent in vivo.

The article in Anticancer Research 8: 1373-1376 (1988), "Antimetastatic Effect of Amiloride in an Animal Tumour Model", by Kellen et al discloses how amiloride that was continuously administered orally to rats inhibited the metastasis of circulating cancer cells into the lung of the rat. The authors suggested that the amiloride prevented tumor cells from migrating through extracellular matrix. In vitro, amiloride competitively inhibited the catalytic activity of uPA on plasminogen.

It is noted that some of the references mentioned above relate to experiments conducted in vitro and some relate to experiments conducted in vivo. Inconsistencies have been noted in FEBS Letters, Vol. 168, pg. 33-37, 1984, "Human Endothelial Cells Contain One Type of Plasminogen Activator" by Kristensen et al between these two types of experiments, and the authors suggested that the discrepancies may be due to the fact that cultured cells are not necessarily representative of cells in the intact organism with respect to plasminogen activator synthesis. From the above, it is clear that amiloride or a pharmaceutically acceptable salt or solvate thereof, is not known as an agent for treating ocular neovascularization, especially corneal, iridial, retinal, and choroidal neovascularization.

## Summary of the Invention

Accordingly, it is an object of the present invention to provide a material to inhibit ocular neovascularization that is a commonly available material whose pharmacological properties and side effects are well known.

Another object of the invention is to provide a material to inhibit ocular neovascularization that can be applied to a patient's eye directly, or by implantable devices, oral administration, or intravenous administration.

Another object of the invention is to provide a material to inhibit retinal neovascularization that can be delivered to a patient's eye directly, or via oral or intravenous administration.

Another object of the invention is to provide a material to inhibit corneal neovascularization that can be delivered to a patient's eye directly, or via oral or intravenous administration.

Still another object of the invention is to provide a material to inhibit ocular neovascularization without being rejected by the body's immune system.

Yet another object of the invention is to provide an ocular treatment that impedes neovascularization that occurs in age-related macular degeneration and diabetic retinopathy.

Another object of the invention is to provide an ocular treatment that impedes neovascularization that occurs in retinal and choroidal tissues.

Yet another object is to provide a treatment for retinal neovascularization that avoids impairment of the patient's peripheral and night vision.

Additional objects, advantages, and novel features of the invention will be set forth in part in the description that follows and in part will become apparent to those skilled in the art upon examination of the following or may be learned with the practice of the invention. The objects and advantages of the invention may be realized and attained by means of the instrumentalities and combinations particularly pointed out in the appended claims.

To achieve the foregoing and other objects, and in accordance with the purposes of the present invention as described herein, an improved method of treating ocular tissues employs the application of an effective amount of amiloride, or a related pyrazine derivative or congener including pharmaceutically acceptable salts and/or solvates thereof, directly or systemically to ocular tissues to inhibit or prevent ocular neovascularization. More specifically, the pyrazine derivatives prevent corneal, iridial, retinal, and choroidal neovascularization.

Still other objects of the present invention will become readily apparent to those skilled in this art from the following description.

## Detailed Description of Preferred Embodiments

Amiloride can be administered to a human patient in the form of an eyedrop solution which includes amiloride or a pharmaceutically acceptable salt or solvate thereof and a carrier such as an artificial tear solution.

An artificial tear carrier solution can be an aqueous buffered isotonic solution.

For purposes of the present invention, the term amiloride-related compound used herein is understood to include free-base forms and/or pharmaceutically acceptable salts and/or solvates of amiloride-related compounds. More specifically, when an off-the-shelf amiloride-related compound or salt and/or solvate is dissolved in an aqueous solution in the presence of a pharmaceutically acceptable buffer, a complex equilibrium mixture is established that includes free-base forms, protonated cationic forms, and anionic forms. Such mixtures are administered to a patient.

Here are represented several formulations for pharmaceutical compositions of the invention. Such formulations, or the like, can be used for preventing corneal, iridial, retinal, and choroidal neovascularization. It is noted that the molecular weight of amiloride is 229.65, and that of amiloride monohydrochloride dihydrate (or amiloride-HCl·2H$_2$O) is 303.14.

### Example I

Approximately 3.03 grams of amiloride-HCl·2H$_2$O are dissolved in an artificial tear solution and are brought to a volume of one liter. This formulation provides an approximately 10 millimolar solution of amiloride-HCl in artificial tear carrier for application to the patient by means of eyedrops. This solution is approximately 0.29% amiloride-HCl by weight.

### Example 2

Approximately 3.33 grams of amiloride-HCl·2H$_2$O are dissolved in an artificial tear solution. Then approximately 10 grams of methylcellulose are also added to the solution. The solution is brought to a volume of one liter with isotonic sodium chloride artificial tear solution. This formulation provides an approximately 11 millimolar solution of amiloride-HCl in artificial tear carrier containing approximately 1% methylcellulose for application to the patient by means of eyedrops. This solution is approximately 0.31% amiloride-HCl by weight.

### Example 3

Approximately 3.64 grams of amiloride-HCl·2H$_2$O are dissolved in an artificial tear solution along with 14 grams of polyvinyl alcohol, and the solution is brought to a volume of one liter with the artificial tear solution. This formulation provides an approximately 12 millimolar solution of amiloride-HCl in artificial tear carrier containing approximately 1.4% polyvinyl alcohol for application to the patient by means of eyedrops. This solution is approximately 0.34% amiloride-HCl by weight.

### Example 4

Approximately 3.94 grams of amiloride-HCl·2H$_2$O are dissolved in an artificial tear solution and are brought to a volume of one liter. This formulation provides an approximately 13 millimolar solution of amiloride-HCl in artificial tear carrier for application to the patient by means of eyedrops. This solution is approximately 0.37% amiloride-HCl by weight.

### Example 5

Approximately 4.24 grams of amiloride-HCl·2H$_2$O are dissolved in an artificial tear solution and are brought to a volume of one liter. This formulation provides an approximately 14 millimolar solution of amiloride-HCl in artificial tear carrier for application to the patient by means of eyedrops. This solution is approximately 0.40% amiloride-HCl by weight.

### Example 6

Another eyedrop formulation is made by blending the following: 4.55 grams amiloride-HCl·2H$_2$O and a volume of isotonic sodium chloride artificial tear solution with stirring to provide a liter of amiloride-HCl solution. This provides a 15 millimolar solution of amiloride. This solution is approximately 0.43% amiloride-HCl by weight.

Generally speaking, the eyedrop formulations are in a concentration of from 10-15 millimolar. Approximately 1-5% of the amiloride in the drop is expected to penetrate into the cornea. Approximately 0.1% of the amiloride in the drop is expected to penetrate into the retina. Penetration into the interior eye tissues can be facilitated by the presence of a surfactant such as benzalkonium chloride which is included in the examples 9-14 below as a bacteriocidal agent.

An ointment can also be formulated for administering the amiloride (per the following examples).

## Example 7

The following ointment can be prepared by blending 3.03 grams of amiloride-HCl·2H$_2$O thoroughly with about 997.0 grams of a petrolatum vehicle (an ointment base) to provide an ointment suitable for application to the ocular tissues which contains approximately 0.29% amiloride-HCl in petrolatum by weight.

## Example 8

The following ointment can be prepared by blending approximately 4.55 grams of amiloride-HCl·2H$_2$O thoroughly with approximately 995.4 grams of petrolatum vehicle (an ointment base) to provide an ointment suitable for application to the ocular tissues which contains approximately 0.43% amiloride-HCl in petrolatum by weight.

Additional eyedrop formulations are presented below:

## Example 9

Approximately 3.03 grams of amiloride monohydrochloride dihydrate are added to an aqueous solution containing 0.0329% (w/v) sodium phosphate monobasic monohydrate, 0.0613% (w/v) sodium phosphate dibasic dihydrate, 0.0040% (w/v) benzalkonium chloride and 2.43% (w/v) glycerin. The pH of the solution is adjusted with 1 N sodium hydroxide or 1 N hydrochloric acid as needed to pH 6.8 and the solution is brought to a final volume of 1 liter by addition of water. This formulation provides an approximately 10 millimolar solution of amiloride monohydrochloride in a buffered isotonic solution for application to the patient by means of eyedrops. The solution is approximately 0.29% amiloride monohydrochloride by weight.

## Example 10

Approximately 3.33 grams of amiloride monohydrochloride dihydrate are dissolved in an aqueous solution containing the components described in Example 9. The pH is adjusted as before and the volume is brought to a final volume of 1 liter by addition of water. This formulation provides an approximately 11 millimolar solution of amiloride monohydrochloride in a buffered isotonic solution for application to the patient by means of eyedrops. The solution is approximately 0.31% amiloride monohydrochloride by weight.

## Example 11

Approximately 3.64 grams of amiloride monohydrochloride dihydrate are dissolved in an aqueous solution containing the components described in Example 9. The pH is adjusted as before and the volume is brought to a final volume of 1 liter by addition of water. This formulation provides an approximately 12 millimoloar solution of amiloride monohydrochloride in a buffered isotonic solution for application to the patient by means of eyedrops. The solution is approximately 0.34% amiloride monohydrochloride by weight.

## Example 12

Approximately 3.94 grams of amiloride monohydrochloride dihydrate are dissolved in an aqueous solution containing the components described in Example 9. The pH is adjusted as before and the volume is brought to a final volume of 1 liter by addition of water. This formulation provides an approximately 13 millimolar solution of amiloride monohydrochloride in a buffered isotonic solution for application to the patient by means of eyedrops. The solution is approximately 0.37% amiloride monohydrochloride by weight.

## Example 13

Approximately 4.24 grams of amiloride monohydrochloride dihydrate are dissolved in an aqueous solution containing the components described in Example 9. The pH is adjusted as before and the volume is brought to a final volume of 1 liter by addition of water. This formulation provides an approximately 14 millimolar solution of amiloride monohydrochloride in a buffered isotonic solution for application to the patient by means of eyedrops. The solution is approximately 0.40% amiloride monohydrochloride by weight.

## Example 14

Approximately 4.55 grams of amiloride monohydrochloride dihydrate are dissolved in an aqueous solution containing the components described in Example 9. The pH is adjusted as before and the volume is brought to a final volume of 1 liter by addition of water. This formulation provides an approximately 15 millimolar solution of amiloride monohydrochloride in a buffered isotonic solution for application to the patient by means of eyedrops. The solution is approximately 0.43% amiloride monohydrochloride by weight.

Instead of a petrolatum-based vehicle, a water soluble gel base can also be used as a carrier for the amiloride.

Other formulations of eyedrops can contain ethylenediamine tetraacetic acid to act as a stabilizer. Hyaluronic acid can also be added to an eyedrop formulation to keep the amiloride-HCl in contact with the eye for a longer time period, or the amiloride can be applied in a collagen or hyaluronic acid gel. As described above, a surfactant such as benzalkonium chloride may be added to the eyedrops to facilitate migration of the amiloride compound from the exterior of the eyeball, where the drops are applied, to the interior tissues of the eye such as the choroid and the retina.

Another form for administering the amiloride can be a contact lens that has been dipped in an amiloride-HCl solution. A contact lens that is dipped in the amiloride-HCl solution can be composed of collagen such that the lens forms a collagen shield. Amiloride can also be administered by polymeric microspheres containing amiloride-HCl.

Another methodology that may be employed for administering the amiloride compound in accordance with the invention is by means of a slow-release implant intrastromally, in the vitreous cavity, or attached to an intraocular lens implant. Such an implant could be placed at the time of ocular surgery, thereby combining it with the surgical procedure. The vitreous implant can be biodegradable, so that when the amiloride compound has been dispensed, the implant disappears.

Still another form of administration of amiloride is by means of an implant in the inferior conjunctiva cul-de-sac.

Yet another mode of administration of amiloride is by oral administration. Intravenous administration can also be employed. Another mode of administration can be by means of an intrascleral implant, especially under the macular region of the retina.

A series of experiments have been conducted employing a rabbit model. Slow release pellets containing a stimulator (e.g. a prostaglandin $E_1$ ($PGE_1$) of neovascularization were implanted in a small slit cut into the respective corneas of 21 rabbits. The implants were pellets made of ethylene vinyl-acetate copolymer. Within 3 days, neovascularization could be clearly detected in the corneas adjacent to the implanted pellets. At this time, amiloride-HCl·$2H_2O$-containing pellets of agarose were implanted in a second slit on one side of each $PGE_1$ pellet slit a prescribed distance from the area of active neovascularization. Blank control agarose pellets were implanted in a similar fashion as controls in third slits on the opposite side of the $PGE_1$ pellet the same prescribed distance from the area of active neovascularization. Amiloride inhibited neovascularization in a dose dependent manner. See Table I below.

## TABLE I

### Effect of Amiloride on Growth of Corneal Vessels

| Dose | N | E/C Day 2 | E/C Day 4 | E/C Day 6 |
|------|---|-----------|-----------|-----------|
| 12 µg | 4 | .80 (±.15) | .95 (±.52) | 1.21 (±.44) |
| 25 µg | 4 | .86 (±.19) | .88 (±.24) | 1.21 (±.35) |
| 50 µg | 4 | .65 (±.21) | .42 (±.20) | .54 (±.21) |
| 100 µg | 5 | .36 (±.13) | .16 (±.05) | .54 (±.10) |
| 400 µg | 3 | .63 (±.13) | .28 (±.20) | .29 (±.13) |
| 500 µg | 1 | .71 (-) | .20 (-) | .21 (-) |

values are means ± SEM

E/C = ratio of Experimental to Control
vessel length

In addition to impeding or inhibiting further neovascularization, amiloride has been observed to even reverse a portion of the neovascularization that had already been produced. Thus, amiloride may be used for treating present cases of neovascularization to reverse the neovascularization.

Beyond preventing further neovascularization occurring in a patient having some degree of neovascularization, and beyond reversing an already developed neovascularization, the amiloride may be administered prophylactically to a patient who may be predisposed to developing ocular neovascularization.

Thus far, only amiloride has been disclosed as being suitable for preventing ocular neovascularization. Amiloride is also known by the following names: 3,5-diamino-6-chloro-N--(diaminomethylene)pyrazinecarboxamide; 3,5-diamino-N--(aminoiminomethy1)-6-chloropyrazinecarboxamide; N-amidino-3,5--diamino-6-chloropyrazinecarboxamide; N-amidino-3,5-diamino-6--chloropyrazinamide; 1-(3,5-diamino-6--chloropyrazinecarboxyl)guanidine; 1-(3,5-diamino-6--chloropyrazinoyl)guanidine; guanamprazine; amipramidin; amipramizide; MK-870; amikal, arumil, colectril, midamor, and modamide. Amiloride is normally used in the form of amiloride monohydrochloride dihydrate.

Although amiloride has been disclosed as a suitable agent for treating ocular tissues to prevent retinal neovascularization, there are a number of other amiloride-related pyrazine derivatives, analogs or congeners which may also be suitable in preventing the neovascularization.

Table II presented below lists amiloride and sixteen derivatives. Table II includes: alphanumeric codes for the amiloride derivatives, analogs or congeners; chemical structures; and common names of the derivatives.

More specifically, the method for prevention of ocular neovascularization includes administration of an amiloride-pyrazine derivative compound that is selected from the group consisting of free-base forms of or pharmaceutically acceptable salts and/or solvates of 3,5-diamino-6-chloro-N--(diaminomethylene)pyrazinecarboxamide also known as amiloride, 3-amino-5-dimethylamino-6-chloro-N--(diaminomethylene)pyrazinecarboxamide also known as 5-(N,N--dimethyl)amiloride or DMA, 3-amino-5-(1-homopiperidinyl)-6--chloro-N-(diaminomethylene)pyrazinecarboxamide also known as 5-(N,N-hexamethylene)amiloride or EIPA, 3-amino-5-(N-ethyl-N--isopropylamino)-6-chloro-N-(diaminomethylene)pyrazinecarboxamide also known as 5-(N-ethyl-N-isopropyl)amiloride or EIPA, 3-amino--5-(N-methyl-N-isobutylamino)-6-chloro-N-(diaminomethylene)pyrazinecarboxamide also known as 5-(N-methyl--N-isobutylamiloride or MIBA, 3,5-diamino-6-chloro-N--(phenylaminoaminomethylene)pyrazinecarboxamide also known as phenamil, 3-amino-5-(N-4-chlorobenzylamino)-6-chloro-N-[(2,4-dimethylbenzyl)aminoaminomethylene]pyrazinecarboxamide also known as 5-(N-4-chlorobenzyl)-2',4'-dimethylbenzamil or CBDMB, 3--amino-5,6-dichloro-N-(diaminomethylene)pyrazinecarboxamide also known as 5-chloroamiloride or 5-ClA, 3,5-diamino-6-fluoro-N-(diaminomethylene)pyrazinecar-

8

boxamide also known as 6-fluoroamiloride or 6-FA, 3-amino-N-(diaminomethylene)pyrazinecarboxamide also known as 5,6-dihydroamiloride or 5,6-HA, 3,5-diamino-6-iodo-N-(diaminomethylene)pyrazinecarboxamide also known as 6-iodoamiloride or 6-IA, 3,5-diamino-N-(diaminomethylene)pyrazinecarboxamide also known as 6-hydroamiloride or 6-HA, 3-amino-5-(N,N-dimethylamino)-6-iodo-N--(diaminomethylene)pyrazinecarboxamide also know as 6-iodo-5--(N,N-dimethyl)amiloride or IDMA, 3-amino-6-bromo-N--(diaminomethylene)pyrazinecarboxamide also known as 6-bromo-5--hydroamiloride or 6-Br-5-HA, 3,5-diamino-6-chloro-N²--(diaminomethylene)pyrazinecarbonyl hydrazide also known as MK--875, 3,5-diamino-6-bromo-N-(diaminomethylene)pyrazinecarboxamide also known as 6-bromoamiloride or 6-BrA, and 3,5-diamino-6--chloro-N-(benzylaminoaminomethylene)pyrazinecarboxamide also known as benzamil, 3-amino-6-chloro-5-[N-methyl-N-(guanidinocarbonylmethylamino)]-N(diaminomethylene)pyrazine carboxamide also known as 5-[N-methyl-N--(guanidinocarbonylmethyl)]amiloride or MGCMA.

It is noted that the pyrazine derivatives in Table II have substituents on the 2-position, 3-position, 5-position, and 6-position of the pyrazine ring. It is further noted that the DMA, HMA, EIPA, MIBA, PHENAMIL, CBDMB, 6-HA, 6-FA, 6-IA, IDMA, 6-BrA, BENZAMIL, and MGCMA pyrazine derivatives in Table II bear a carboxamide substituent in the 2-position, wherein the carboxamide moiety bears a nitrogen atom bearing a - (aminoiminomethyl), -(arylaminoiminomethyl), - (aralkylaminoiminomethyl), or -[(substituted- aralkyl)aminoiminomethyl] group. It is further noted that the pyrazine derivative DMA, HMA, EIPA, MIBA, PHENAMIL, CBDMB, 6-FHA, 6-HA, 6-IA, IDMA, 6-BrA, BENZAMIL, and MGCMA in Table II bear an amino substituent in the 3-position, an amino or substituted amino substituent in the 5-position, and a halogeno substituent or a hydrogen in the 6-position.

Table III lists amiloride and the sixteen derivatives listed in Table II. Table III also lists the relative inhibition of uPA for a 100 micromolar dose and the relative inhibition for a 20 micromolar dose.

## Table III

### % INHIBITION OF uPA ACTIVITY

| COMPOUND | 20 mM INHIBITOR | 100 mM INHIBITOR |
|---|---|---|
| AMILORIDE | 20.5 | 46.6 |
| DMA | 41.1 | 48.3 |
| HMA | 51.1 | 77.0 |
| EIPA | 28.3 | 56.0 |
| MIBA | 18.7 | 57.3 |
| PHENAMIL | 1.7 | 5.5 |
| CBDMB | 41.7 | 63.4 |
| 5-ClA | 5.0 | 20.7 |
| 6-FA | 7.1 | 27.9 |
| 5,6-HA | 2.1 | 11.1 |
| 6-IA | 64.4 | 83.9 |
| 6-HA | 15.3 | 38.2 |
| IDMA | 40.3 | 73.1 |
| 6-Br-5-HA | 1.0 | 7.7 |
| MK-875 | 1.9 | 7.3 |
| 6-BrA | 65.6 | 69.9 |
| BENZAMIL | 1.2 | 7.7 |
| MGCMA | 29.1 | 43.0 |

The values for the relative inhibitions contained in Table III are obtained by the following procedure.

An in vitro assay employs a commercially available synthetic chromogenic substrate, glutaryl-glycyl-arginyl-p-nitroanilide, and a commercially available human uPA. The uPA catalyzes the hydrolysis of the bond between the arginyl and p-nitroanilide moieties. The p-nitroaniline released upon hydrolysis of the substrate absorbs light at a wavelength of 405nm. The enzymatic activity is quantitated as a function of the increase in the absorbance of the solution at 405nm as measured with a spectrophotometer.

With respect to the data in Table III, the number in the table is the percentage of inhibition of release of p-nitroaniline from the substrate observed upon inclusion of the respective compound at the concentration noted in the uPA assay. The greater the number in the table, the greater the ability of the amiloride-related pyrazine derivative compound to inhibit uPA activity. In order to obtain the data, stock solutions of the amiloride-related pyrazine derivative compounds were included in the assays, and the release of p-nitroaniline as followed by the change in absorbance at 405nm of the reaction mixture was compared to a control reaction mixture in which no amiloride-related pyrazine derivative compound was present.

Although the phenomena occurring with the amiloride-related pyrazine derivative ocular treatment of the invention to prevent retinal neovascularization are not fully understood, a theoretical explanation is provided herein in order to lend greater understanding as to the operation of the invention. Briefly, the main body of an already-formed capillary has, from inside to outside, an inner luminal surface, vascular endothelial cells, an abluminal surface, and a basement membrane. A capillary has a single-cell tip which penetrates into extracellular matrix when the capillary grows. The capillary actually grows longitudinally from behind the tip when cell division occurs as the tip invades the surrounding matrix and tissue. At the tip of the newly growing capillaries, urokinase type plasminogen activator (uPA) is present. UPA is capable of activating enzymes which degrade the extracellular matrix components, and thus allow cell penetration into surrounding tissue. Once surrounding tissue is penetrated, the new vessel matures into an established vessel. The maturation process involves formation of intercellular contacts, creation of luminal and abluminal endothelial cell surfaces, and recreation of a surrounding specialized extracellular matrix (basement membrane). With this invention, it has been discovered that amiloride-related pyrazine derivative materials appear to inhibit the operation of uPA such that ocular neovascularization is inhibited. Generation of plasmin, cleavage of peptide substrates, and the interaction of uPA with a specific macromolecular proteinase substrate in ocular tissues are all prevented in the presence of amiloride. In contrast, Vassalli and Belin report that amiloride does not inhibit tissue-type plasminogen activator (tPA), plasmin, plasma kallikrein or thrombin in vitro. The specificity of amiloride for uPA inhibition over tPA inhibition has important therapeutic implications. Whereas uPA is involved in extracellular matrix degradation during cell migration, tPA is involved in the destruction of fibrin clots, a process critical to an organism's hemostatic balance. Inappropriate inhibition of tPA could have dangerous side effects.

In summary, numerous benefits have been described which result from employing the principles of the invention. With the invention, an amiloride composition is provided for inhibiting ocular neovascularization. Amiloride-HCl·2H$_2$O is a readily available material which has been studied extensively for its pharmacological properties as a diuretic, and side effects from use as a diuretic are well known. With the invention, a composition containing amiloride-HCl is provided that can be applied to a patient's eye directly. The amiloride-HCL can also be administered orally. The amiloride-related composition is useful in preventing corneal, iridial, retinal, and choroidal neovascularization.

In addition, with the invention, an ocular treatment is provided that can be used to prevent neovascularization that occurs in age-related macular degeneration and diabetic retinopathy.

By employing the invention, a topical inhibitor of ocular neovascularization is provided which can improve the post-operative management of corneal transplants. With the invention, there is provided an ocular treatment that prevents neovascularization that occurs in retinal and choroidal tissues. By employing the invention, a treatment for retinal neovascularization is provided that avoids impairment of the patient's peripheral and night vision.

The foregoing description of the invention has been presented for purposes of illustration and description. It is not intended to be exhaustive or to limit the invention to the precise form disclosed. Obvious modifications or variations are possible in light of the above teachings. The embodiments were chosen and described in order to best illustrate the principles of the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. A pharmaceutical composition for preventing ocular neovascularization characterized by:
      an amount of a compound in the class of pyrazine derivatives, and/or its pharmaceutically acceptable salts, and/or solvates effective to prevent ocular neovascularization in an animal or human patient; and
      a carrier for said pyrazine derivatives and/or pharmaceutically acceptable salts, and/or solvates, said carrier suitable for topical application to ocular tissues.

2. The pharmaceutical composition described in claim 1, further characterized by said pyrazine derivative having a substituent on the 3-position, 5-position, and 6-position on the pyrazine ring,
      wherein said pyrazine derivative bears a carboxamide substituent in the 2-position, and the carboxamide moiety nitrogen atom bears a -(aminoiminomethyl), - (arylaminoiminomethyl), -(aralkylaminoiminomethyl), or - [(substituted-aralkyl) aminoiminomethyl] group,

wherein said pyrazine derivative bears an amino substituent in the 3-position,

wherein said pyrazine derivative bears an amino or substituted amino substituent in the 5-position, and

wherein maid pyrazine derivative bears a halogeno substituent or a hydrogen in the 6-position, and pharmaceutically acceptable salts and/or solvates.

3. The pharmaceutical composition described in claim 1, further characterized by said pyrazine derivative being amiloride, and/or its pharmaceutically acceptable salts and/or solvates.

4. The pharmaceutical composition described in claim 1, Further characterized by said pyrazine derivative being selected from the group consisting of free-base forms of or pharmaceutically acceptable salts and/or solvates of 3,5-diamino-6-chloro-N--(diaminomethylene)pyrazinecarboxamide also known as amiloride, 3-amino-5-dimethylamino-6-chloro-N--(diaminomethylene)pyrazinecarboxamide also known as 5-(N,N--dimethyl)amiloride or DMA, 3-amino-5-(1-homopiperidinyl)-6--chloro-N-(diaminomethylene)pyrazinecarboxamide also known as 5--(N,N-hexamethylene)amiloride or HMA, 3-amino-5-(N-ethyl-N--isopropylamino)-6-chloro-N-(diaminomethylene)pyrazinecarboxamide also known as 5-(N-ethyl-N-isopropyl)amiloride or EIPA, 3-amino--5-(N-methy1-N-isobutylamino)-6-chloro-N--(diaminomethylene)pyrazinecarboxamide also known as 5-(N-methyl--N-isobutylamiloride or MIBA, 3,5-diamino-6-chloro-N--(phenylaminoaminomethylene)pyrazinecarboxamide also known as phenamil, 3-amino-5-(N-4-chlorobenzylamino)-6-chloro-N-[(2,4--dimethylbenzyl)aminoaminomethylene]pyrazinecarboxamide also known as 5-(N-4-chlorobenzyl)-2',4'-dimethylbenzamil or CBDMB, 3,5-diamino-6-fluoro-N--(diaminomethylene)pyrazinecarboxamide also known as 6-- fluoroamiloride or 6-FA, 3,9-diamino-6-iodo-N--(diaminomethylene)pyrazinecarboxamido also known as 6--iodoamiloride or 6-IA, 3,5-diamino-N--(diaminomethylene)pyrazinecarboxamide also known as 6-hydroamiloride or 6-HA, 3-amino-9-(N,N-dimethylamino)-6-iodo-N--(diaminomethylene)pyrazinecarboxamide also know as 6-iodo-5--(N,N-dimethyl)amiloride or IDMA, 3,5--diamino-6-bro-mo-N-(diaminomethy-lene)pyrazinecarboxamide also known am 6-bromoamiloride or 6-BrA, 3,5-diamino-6--chloro-N-(benzylaminoaminomethy)ene)pyrazinecarboxamide also known as benzamil, 3-amino-6-chloro-5-[N-methyl-N--(guanidinocarbonylmethylamino)]-N-(diaminomethylene)pyrazine carboxamide also known as 5-[N-methyl-N--(guanidinocarbonylmethyl)]amiloride or MGCMA.

5. The pharmaceutical composition described in claim 1, further characterized by said carrier being in a form selected from the group consisting of a buffered isotonic composition, an ointment base, a water soluble gel, and an artificial tear composition.

6. The pharmaceutical composition described in claim 5, further characterized by maid artificial tear composition including a cellulose ether selected from the group consisting of methylcellulose, hydroxypropylmethylcellulose, and hydroxyethylcellulose.

7. The pharmaceutical composition described in claim 5, further characterized by said artificial tear composition including polyvinyl alcohol.

8. The pharmaceutical composition described in claim 1, further characterised by said pyrazine derivative, and/or its pharmaceutically acceptable salts and/or solvates, being prevent in an amount effective to inhibit types of ocular neovascularization selected from the group consisting of retinal neovascularization, corneal neovascularization, choroidal neovascularization, and iris neovascularization.

9. A method of treating a human being for inhibition, reduction, or prevention of ocular neovascularization, characterized by the steps of: administering to the human being an amount of a compound in the class of pyrazine derivatives, and/or its pharmaceutically acceptable salts and/or solvates, effective to inhibit, reduce, or prevent ocular neovascularization.

10. The method described in claim 9, further characterized by the compound in the class of pyrazine derivatives, and/or its pharmaceutically acceptable salts and/or solvates, being administered by direct application to ocular tissues.

11. The method described in claim 9, further characterized by the compound in the class of pyrazine derivatives, and/or its pharmaceutically acceptable salts and/or solvates, being administered by oral administration.

12. The method described in claim 9, further charactarized by the compound in the class of pyrazine derivatives, and/or its pharmaceutically acceptable salts and/or solvates, being administered by intraocular implant.

13. The method described in claim 9, further characterized by the compound in the class of pyrazine derivataves, and/or its pharmaceutically acceptable salts and/or solvates, being selected from tho group consisting of free-base forms or pharmaceutically acceptable salts and/or solvates of 3,5-diamino-6-chloro-N--(diaminomethelene)pyrazinecarboxamide also known as amiloride, 3-amino-5-dimethylamino-6-chloro-N--(diaminomethylene)pyrazinecarboxamide also known as 5-(N,N--dimethyl)amiloride or DMA, 3-amino-5-(1-homopiperidinyl)-6--chloro-N-(diaminomethylene)pyrazinecarboxamide also known as 5--(N,N-hexamethylene)amiloride or HMA, 3-amino-9-(N-ethyl-N--isopropylamino)-6-chloro-N-(diaminomethylene)pyrazinecarboxamide also known as 5-(N-ethyl-N-isopropyl)amiloride or MIPA, 3-amino--5-(N-methy1-N-isobutylamino)-6-chloro-N--(diaminomethylene)pyrazinecarboxamide also known as 5-(N-methyl--N-isobutylamiloride or MIBA, 3,5-diamino-6-chloro-N--(phenylaminoaminomethylene)pyrazinecarboxamide also known as phenamil, 3-amino-5-(N-4-chlorobenzylamino)-6-chloro-N-[(2,4---dimethylbenzyl)aminoaminomethylene]pyrazinecarboxamide also known as 5-(N-4-chlorobenzyl)-2',4'-dimethylbenzamil or CBDMB, 3,5-diamino-6-fluoro-N--(diaminomethylene)pyrazinecarboxamide also known as 6--fluoroamiloride or 6-FA,, 3,5-diamino-6-iodo-N--(diaminomethylene)pyrazinecarboxamide also known as 6--iodoamiloride or 6-IA, 3,5-diamino-N--(diaminomethylene) pyrazinecarboxamide also known as 6--hydroamiloride or 6-HA, 3-amino-5-(N,N-dimethylamino)-6-iodo-N--(diaminomethylene)pyrazineoarboxamide also know as 6-iodo-6--(N,N-dimethyl)amiloride or IDMA, 3,5-diamino-6-bromo-N-(diaminomethylene)pyrazinecarboxamide also known as 6-bromoamiloride or 6-BrA, 3,5-diamino-6-chloro-N-(benzylaminoaminomethylene)pyrazinecarboxamide also known as benzamil, and 3-amino-6-chloro-5-[N-methyl-N--(guanidinocarbonylmethylamino)]-N-(diaminomethylene) pyrazine carboxamide also known as 5-[N-methyl-N--(guanidinocarbonylmethyl)]amiloride or MGCMA.

14. The method described in claim 9, further characterized by the pyrazine derivative, and/or its pharmaceutically acceptable salts and/or solvates, being present in an amount effective to inhibit, reduce, or prevent types of ocular neovascularization selected from the group consisting of retinal neovascularization, corneal neovascularazation, choroidal neovascularization, and iris neovascularization.

15. A method of treating a human being for inhibition, reduction, or prevention of ocular neovascularization, characterized by the steps of: administering to the human being an amount of a compound which inhibits urokinase-like plasminogen activator (uPA), the compound being administered by oral administration, the compound being administered in an amount effective to inhibit, reduce, or prevent ocular neovascularization.